Europäisches Patentamt

European Patent Office

Office européen des brevets

(19)

(11) Numéro de publication: **0 210 091**
**B1**

(12) **FASCICULE DE BREVET EUROPEEN**

(45) Date de publication du fascicule du brevet: **25.04.90**

(21) Numéro de dépôt: **86401345.3**

(22) Date de dépôt: **19.06.86**

(51) Int. Cl.⁵: **A 61 K 49/02,** G 21 H 5/02, G 01 N 33/60

(54) **Procédé nouveau de réduction du pertechnétate.**

(30) Priorité: **05.07.85 FR 8510308**

(43) Date de publication de la demande:
**28.01.87 Bulletin 87/05**

(45) Mention de la délivrance du brevet:
**25.04.90 Bulletin 90/17**

(84) Etats contractants désignés:
**AT BE CH DE FR GB LI LU NL SE**

(56) Documents cités:
**EP-A-0 007 676**
**EP-A-0 038 546**
**EP-A-0 096 871**
**GB-A-2 082 321**
**US-A-4 058 593**

(73) Titulaire: **Causse, Jean-Etienne**
**Résidence "Les Launes" Bâtiment 2 Impasse Tour Buffel**
**F-34100 Montpellier (FR)**

(72) Inventeur: **Causse, Jean-Etienne**
**Résidence "Les Launes" Bâtiment 2 Impasse Tour Buffel**
**F-34100 Montpellier (FR)**

(74) Mandataire: **Hirsch, Marc-Roger**
**Cabinet Hirsch 34 rue de Bassano**
**F-75008 Paris (FR)**

Courier Press, Leamington Spa, England.

EP 0 210 091 B1

## EP 0 210 091 B1

**Description**

La présente invention a pour objet un procédé de réduction de technétium et d'obtention de technétium à l'état d'oxydation réduit.

En fait, la présente invention concerne la production de technétium réduit à partir de pertechnétate de sodium et se rapporte, en outre, au technétium réduit ainsi obtenu, aux compositions destinées à être employées dans les techniques de marquage radio-médical les contenant ainsi qu'aux procédés de marquage radio-médical, par exemple de scintigraphies utilisant de telles compositions.

Par "technétium réduit", on entend dans la présente description et dans les revendications auxquelles elle donne lieu du technétium à l'état libre ou combiné dont l'état d'oxydation est inférieur à l'état d'oxydation du technétium dans le pertechnétate de sodium.

On sait que dans les techniques de scintigraphies ou autres techniques de radio-pharmacie est fait très largement emploi, en tant que radio-isotope, de l'isotope de poids moléculaire 99 métastable (dit 99m) du technétium, lequel se transforme en émettant un rayonnement γ pur, sans autre radiation, dont l'énergie est de 140 KEV. Sa période est de six heures. L'intérêt de ce radio-isotope en médecine nucléaire est quadruple:

— il est aisément détectable du fait que son rayonnement permet une pénétration suffisante des tissus;

— il est aisément collimatable, du fait qu'il donne une résolution excellente pour les gamma-caméras;

— des quantités importantes peuvent en être administrées (de l'ordre du millicurie) du fait de l'absence de rayonnement α ou β parasites;

— sa période de désintégration est voisine de six heures.

Cependant, pour obtenir la meilleure répartition du technétium dans les régions de l'organisme que l'on veut observer, il est nécessaire que le technétium soit employé sous forme de complexes stables, ce qui nécessite l'emploi de technétium sous le degré d'oxydation correspondant à la valence 7 et à l'état de chélates qui ne se forment qu'avec les dérivés réduits du technétium.

Pour obtenir le technétium sous cette forme la plus avantageuse, diverses méthodes ont été proposées. L'une d'elles fait appel à une réduction par le chlorure stanneux et l'autre à une réduction par le mélange acide ascorbique/chlorure ferrique.

Ces procédés de réduction connus cependant présentent de nombreux inconvénients:

— le produit de la réduction présente une très faible solubilité à pH supérieur à 5 dans les solutions destinées à être employées en scintigraphie; il en résulte qu'il est nécessaire de vérifier avec soin le pH des solutions destinées à être injectées et de prendre des précautions diverses telles que addition de tampons et autres, pour que ces solutions puissent satisfaire aux exigences de pH physiologique (de l'ordre de 6,9 à 7,4) requises en pharmacopée;

— le produit de la réduction contient des métaux lourds tels que Sn, ayant une action toxique élevée sur les humains tant en soi que par effet cumulatif avec d'autres métaux lourds.

En fait, ces proportions de Sn dans les solutions obtenues dépassent, en général, les niveaux tolérables, et il convient donc de les employer sous contrôle étroit.

La présente invention permet d'obvier les inconvénients des procédés de réduction connus du pertechnétate de sodium et donc d'éviter la présence de métaux lourds tels Sn dans le technétium réduit obtenu et, par conséquent, dans les solutions destinées à être employées en scintigraphie. En fait, le procédé selon l'invention permet d'obtenir un technétium réduit exempt de toute trace d'élément toxique d'une part et d'autre part actif à pH physiologique.

La présente invention a pour objet un procédé de réduction du pertechnétate de sodium et d'obtention de technétium réduit par mise au contact du pertechnétate de sodium avec un système réducteur solide à base de phénol et/ou d'acide maléique associé à un support polymère ou copolymère inerte ou réagissant avec ces composés ou encore sous forme polymérisée ou copolymérisée.

Selon une forme d'exécution de l'invention, le système réducteur solide consiste en un polymère ou copolymère à base de phénol ou d'acide maléique.

Selon une autre forme d'exécution de l'invention, le technétium est l'isotope 99m.

La présente invention a également pour objet les composés à base de technétium réduit et exempts de métaux lourds, tels Sn, et, plus particulièrement, à base d'isotope 99m du technétium obtenus à l'état réduit à l'aide du procédé précité.

La présente invention a, en outre, pour objet les compositions destinées à être utilisées dans les techniques de marquage radio-médicales, contenant comme agent de marquage du 99m-Tc réduit exempts de métaux lourds, tel Sn obtenu à l'aide du procédé précité.

De préférence, le procédé 99m-Tc réduit est obtenu in situ dans lesdites compositions et est à l'état naissant.

Exemple 1

Réduction en milieu neutre du $TcO_4^-$ et application au marquage de l'éthambutol.

L'addition d'une molécule d'eau au copolymère anhydrique maléique styrène lui confère des propriétés réductrices. 100 mg dudit copolymère sont hydrolysés par addition de 1 ml de soude 10N jusqu' à dissolution complète du polmère et reprécipitation par la même quantité d'HCl 10N puis lavés avec 5 volumes d'eau distillée, décantés et filtrés. On constate qu'à pH=7, ces 100 mg de copolymère réduisent instantanément 53 millicuries de pertechnétate de sodium en des composés dans lesquels l'état d'oxydation du 99m-Tc est réduit.

Exemple 2

94 g de phénol pur et 45 g de formol en solution aqueuse à 37% sont mélangés et additionnés de soude 10N jusqu'à l'obtention d'un pH=14.

Après réchauffage prolongé au bain de sable, est obtenue une résine phénol formol insoluble.

1 g de cette résine est mis au contact de 1 millicurie de pertechnétate de sodium à une température de 25°C en présence d'eau. On opère selon la méthode décrite dans l'exemple 1.

Ce traitement donne lieu à la réduction du pertechnétate de sodium en des composés dans lesquels l'état d'oxydation du 99m-Tc est réduit.

Exemple 3

10 g de poudre du polymère ABS (acrylonitrile/butadiène/styrène) sont dissous dans 10 ml de méthyléthylcétone. On ajoute au mélange 0,5 g de 2,6-di-tertiobutyl-4-méthyl-phénol. Après évaporation du solvant par traitement à une température de 60°C, on obtient un bloc de polymère dans lequel le phénol est inclus. Après pulvérisation de ce bloc, on met 1 g de la poudre obtenue au contact de 1 millicurie de pertechnétate de sodium à une température de 25°C. On obtient ainsi des composés dans lesquels l'état d'oxydation du 99m-Tc est réduit.

Exemple 4

Des billes de copolymère anhydride maléique/styrène de 3 mm de diamètre sont formées à l'aide d'une presse à injection. Ces billes sont plongées cinq minutes dans une solution de soude 10N puis dans une solution d'HCl 10N et, enfin, sont lavées à l'aide d'eau distillée. Une bille mise au contact de 1 millicurie de pertechnétate de sodium produit la réduction de ce composé en des composés dans lesquels l'état d'oxydation du 99m-Tc est réduit.

Exemple 5

On dissout 10 g de polyamide type Nylon dans un mélange solvant contenant 20 millilitres de méthyl éthyl cétone et 30 millilitres de dimethylformamide. On ajoute ensuite à ce mélange 10 g d'anhydride maléïque. Après évaporation du solvant par traitement à une température de 60°C, on obtient un bloc de polmère dans lequel l'anhydride maléïque est inclus. Après pulvérisation on obtient une poudre, 1 g de cette poudre réduit 1 millicurie de pertechnétate de sodium.

Exemple 6

On fait fondre 10 g d'anhydride maléïque par chauffage adéquat au bain de sable. On ajoute 1 g de peroxyde de benzoyle et on immerge 5 billes de polystyrène de 3 millimètres de diamètre dont la surface a été érodée mécaniquement. On laisse en contact 5 minutes. On élimine l'excès d'anhydride maléïque par décantation. Après séchage 18h à 25°C, une bille réduit instantanément 1 millicurie de pertechnétate de sodium.

Exemple d'utilisation 1

a. — Une première solution classique destinée à être injectée par voie intraveineuse aux fins d'effectuer la mesure de la fixation rénale d'un patient présente la composition suivante:

| | |
|---|---|
| . acide dimercaptosuccinique | 1,00 mg |
| . chlorure stanneux dihydraté | 0,36 mg |
| . inositol | 50,00 mg |
| . acide ascorbique | 0,70 mg |
| . 99m-Tc-pertechnétate de odium (activité comprise entre 1 et 2 m Ci) | 4 ml |

Le pH de la composition est de l'ordre de 3.

b. — Une deuxième solution selon l'invention destinée au même type de détermination est obtenue par addition de:

| | |
|---|---|
| . acide dimercaptosuccinique | 1,00 mg |
| . inositol | 50,00 mg |

. bille de copolymère acide maléique/styrène:

| | |
|---|---|
| — poids | 0,01 g |
| — diamètre | 3 mm |

. 99m-Tc-pertechnétate de sodium      4 ml

Le pH de la composition ainsi obtenue est de 7.

La bille, bien évidemment, n'est pas destinée à être injectée.

Les deux solutions ci-dessus permettent d'obtenir des scintigraphies ou des images à haute résolution du cortex rénal et d'identifier d'éventuelles lésions tumorales.

En fait, le deuxième solution (b) a un pH neutre, et est donc physiologiquement acceptable, et ne contient ni Sn, ni acide ascorbique dont la présence est gênante.

Les deux solutions conduisent à des résultats comparables du point de vue qualitatif.

Exemple d'utilisation 2

a. — Une première utilisation classique destinée à être injectée par voie intraveineuse est employée pour la scintigraphie osseuse par le pyrophosphate. Il s'agit d'un examen de routine indispensable pour tout cancer "ostéophile". Le double avantage de cette solution est l'exploration de l'ensemble du squelette par un seul examen et sa relative simplicité. Elle permet aussi la détection scintigraphique des infarctus aigus du myocarde et la visualisation des plexus chloroïdes; elle présente la composition suivante:

. pyrophosphate de sodium décahydraté 100    mg

. chlorure stanneux dihydraté       1,6 mg

. pertechnétate de sodium       11,5 mg

(activité comprise entre 6 à 15 m Ci)

. eau stérile       3    ml

Le pH de la solution est de l'ordre de 6.

b. — Une deuxième solution selon l'invention, destinée aux mêmes fins que dans le point (a) ci-dessus, est obtenue par addition de:

. pyrophosphate de sodium décahydraté 100    mg

. bille de copolymère acide maléique/sytrène:

| | |
|---|---|
| — poids | 0,01 g |
| — diamètre | 3    mm |

. 99m-Tc-pertechnétate de sodium      3    ml

Le pH de la solution est de 7.

On constate que la solution (b) a un pH neutre et ne contient pas de métal lourd.

Les deux solutions conduisent à des résultats comparables du point de vue qualitatif.

Exemple d'utilisation 3

L'éthambutol est employé classiquement pour la scintigraphie des poumons et la mise en évidence de tumeurs de cerveaux.

a. — Une première solution est formée de:

. éthambutol       15    mg

. 99m-Tc-pertechnétate de sodium       2    mg

(activité 1 à 10 m Ci)

. chlorure stanneux dihydraté       0,36 mg

b. — Une deuxième solution est obtenue par addition de:

| | | |
|---|---|---|
| . éthambutol | 15 | mg |
| . 99m-Tc-pertechnétate de sodium | 2 | mg |
| . bille de copolymère acide maléique/styrène: | | |
| — poids | 0,01 | g |
| — diamètre | 3 | mm |

On constate que le rendement de marquage avec la solution (a) est de 7%, ce qui est nettement inférieur aux 70% de marquage nécessaires pour les opérations de scintigraphies. La solution (b) permet l'obtention d'un marquage de 75%.

**Revendications pour les Etats contractants: BE CH DE FR GB LI LU NL SE**

1. Procédé de réduction du pertechnétate de sodium et d'obtention de technétium à l'état d'oxydation réduit par mise au contact du pertechnétate de sodium avec un système réducteur solide à base de phénol et/ou d'acide maléique associé à un support polymère ou copolymère inerte ou réagissant avec ces composés ou encore sous forme polymérisée ou copolymérisée.

2. Procédé selon la revendication 1, caractérisé en ce que le système réducteur solide consiste en un polmère ou copolymère à base de phénol ou d'acide maléique.

3. Procédé selon la revendication 1, caractérisé en ce que le technétium est l'isotope 99m.

4. Composés du 99m-Tc réduit obtenu à l'aide du procédé selon une quelconque des revendications 1 à 3, caractérisés en ce qu'ils consistent en des composés du 99m-Tc réduit, exempts de métaux lourds, tel Sn.

5. Compositions destinées à être utilisées dans la technique de marquage radio-médicale, caractérisées en ce qu'elles contiennent des composés selon la revendication 4.

**Revendications pour l'Etat contractant: AT**

1. Procédé de réduction du pertechnétate de sodium et d'obtention de technétium à l'état d'oxydation réduit par mise au contact du pertechnétate de sodium avec un système réducteur solide à base de phénol et/ou d'acide maléique associé à un support polymère ou copolymère inerte ou réagissant avec ces composés ou encore sous forme polymérisée ou copolymérisée.

2. Procédé selon la revendication 1, caractérisé en ce que le système réducteur solide consiste en un polmère ou copolymère à base de phénol ou d'acide maléique.

3. Procédé selon la revendication 1, caractérisé en ce que le technétium est l'isotope 99m.

4. Procédé d'obtention de compositions destinées à être utilisées dans les techniques de marquage radio-médicales, caractérisées en ce qu'elles contiennent des composés du 99m-Tc réduit, exempts de métaux lourds, tel Sn, obtenus par le procédé selon l'une quelconque des revendications 1 à 3.

**Patentansprüche für die Vertragsstaaten: BE CH DE RF GB LI LU NL SE**

1. Verfahren zur Reduktion von Natriumpertechnetat und zur Darstellung von Technetium im reduzierten Oxydationszustand, bei dem Natriumpertechnetat in Berührung gebracht wird mit einem festen Reduktionssystem auf Basis von Phenol und/oder Maleinsäure in Anwesenheit eines polymeren oder kopolymeren Trägers, der inert ist oder mit diesen Verbindungen reagiert oder in polymerisierter oder kopolymerisierter Form vorliegt.

2. Verfahren nach Anspruch 1, dadurch gekennzeichnet, dass das feste Reducktionssystem aus einem Polymerisat oder Kopolymerisat auf Phenol- oder Maleinsäurebasis besteht.

3. Verfahren nach Anspruch 1, dadurch gekennzeichnet, dass das Technetium als Isotop 99m vorliegt.

4. Verbindungen des reduzierten 99cm-Tc, hergestellt nach dem Verfahren gemäss einem der Ansprüche 1 bis 3, dadurch gekennzeichnet, dass sie aus reduzierten, von Schwermetallen wie Sn freien Verbindungen des 99m-Tc bestehen.

5. Zusammensetzungen zur Verwendung auf dem Gebiet der röntgenmedizinischen Markierung, dadurch gekennzeichnet, dass sie Verbindungen nach Anspruch 4 enthalten.

**Patentansprüche fur den Vertragsstaat: AT**

1. Verfahren zur Reduktion von Natriumpertechnetat und zur Darstellung von Technetium im Reduzierten Oxydationszustand, bei dem Natriumpertechnetat in Berührung gebracht wird mit einem festen Reduktionssystem auf Basis von Phenol und/oder Maleinsäure in Anwesenheit eines polymeren oder kopolymeren Trägers, der inert ist oder mit diesen Verbindungen reagiert oder in polymerisierter oder kopolymerisierter Form vorliegt.

2. Verfahren nach Anspruch 1, dadurch gekennzeichnet, dass das feste Reduktionssystem aus einem Polymerisat oder Kopolymerisat auf Phenol- oder Maleinsäurebasis besteht.

3. Verfahren nach Anspruch 1, dadurch gekennzeichnet, dass das Technetium als Isotop 99m vorliegt.

4. Verfahren zur Herstellung von Zusammensetzungen zur Verwendung auf dem Gebiet der röntgenmedizinischen Markierung, dadurch gekennzeichnet, das sie Verbindungen des nach dem Verfahren gemäss einem der Ansprüche 1 bis 3 hergestellten, reduzierten und von Schwermetallen wie Sn freien 99m-Tc enthalten.

**Claims for the Contracting States: BE CH DE FR GB LI LU NL SE**

1. Process for the reduction of sodium pertechnetate and obtaining technetium in the reduced oxydation state by contacting sodium pertechnetate with a solid reduction system based on phenol and/or maltic acid associated with a polymer or copolymer vehicle that is inert or reactive with these compounds or in a polymerized or a copolymerized form.

2. Process according to claim 1, characterized in that the solid reduction system consists of a phenol based or maltic acid based polymer or copolymer.

3. Process according to claim 1, characterized in that the technetium is the isotope 99m.

4. Compounds of reduced 99m-Tc, produced by means of a process according to one of the claims 1 to 3, characterized in that they consist of compounds of reduced 99m-Tc, free from heavy metals such as Sn.

5. Preparations for use in the field of radio-medical marking, characterized in that they contain compounds according to claim 4.

**Claims for the Contracting State: AT**

1. Process for the reduction of sodium pertechnetate and obtaining technetium in the reduced oxydation state by contacting sodium pertechnetate with a solid reduction system based on phenol and/or maltic acid associated with a polymer or copolymer vehicle that is inert or reactive with these compounds or in a polymerized or a copolymerized form.

2. Process according to claim 1, characterized in that the solid reduction system consists of a phenol based or maltic acid based polymer or copolymer.

3. Process according to claim 1, characterized in that the technetium is the isotope 99m.

4. Process for obtaining preparations for use in the field of radio-medical marking, characterized in that they contain compounds of reduced 99m-Tc, free from heavy metals such as Sn, obtained by means of a process according to any one of the claims 1 to 3.